# EUROPEAN PATENT APPLICATION

(11) **EP 4 289 342 A1**
(43) Date of publication of application: **13.12.2023**
(21) Application number: 22182167.1
(22) Date of filing: 30.06.2022
(51) Int. Cl.: A61B 5/00, A61B 6/04, A61B 5/055, G01R 33/28

(54) **A MIRROR SYSTEM FOR RELAYING VIDEO CONTENT TO A SCANNER PATIENT, AND A PATIENT SUPPORT, MEDICAL SCANNER AND PATIENT ENTERTAINMENT SYSTEM USING THE MIRROR SYSTEM**

(30) Priority: 07.06.2022 WO PCT/CN2022/097376
(71) Applicant: Koninklijke Philips N.V., 5656 AG Eindhoven (NL)
(72) Inventor: POSSANZINI, Cecilia, Eindhoven (NL); ADRIAENSSEN, Lieven, Eindhoven (NL); DENG, wen, 5656AG Eindhoven (NL); SENGUPTA, Shubharthi, 5656AG Eindhoven (NL)
(74) Representative: Philips Intellectual Property & Standards

(57) **Abstract**

A mirror system is provided for relaying video content to a patient on a patient support of a medical scanner. The mirror system comprises at least a flexible support arm with first and second opposite ends and a mirror attached to the support arm. The mirror system also comprises at least first and second support members, each for mounting in a position which is fixed relative to the patient support, and each comprising an end portion. The first and second ends of the support arm are each detachably and rotatably couplable to a respective one of the end portions of the support members, and when the support arm is coupled to the end portions it forms an arch.

## Description

### FIELD OF THE INVENTION

The present disclosure relates generally to systems for delivering entertainment to patients in a medical scanner and, in particular, to systems for relaying video content to patients within a medical scanner via a mirror.

### BACKGROUND OF THE INVENTION

Medical scanners are important in clinical settings for diagnosis, treatment planning and treatment monitoring. Generally, a patient is required to lie on a scanner bed, which is then positioned within a bore of the scanner before image acquisition is performed. These scans can be lengthy and noisy, with patients often required to lie relatively still within the scanner bore. In addition, scanner bores may be fairly narrow such that there is little separation between the patient's body and the inner surface of the scanner bore.

Unsurprisingly, patients often suffer from feelings of claustrophobia and anxiety when undergoing tests in these scanners. Providing entertainment to a patient within the scanner can help to distract and relax the patient. This can help to improve the patient's experience during the medical procedure, and potentially improve scan quality by helping to reduce patient restlessness - and thereby patient movement. Accordingly, there is a need to provide systems for entertaining patients within medical scanners.

In addition, it is challenging for clinicians to take physiological measurements from a patient situated in the scanner bore. In particular, clinicians may be interested in reading the patient's vital signs whilst they are being scanned. For example, remote photoplethysmography imaging is a recent emerging technology capable of measuring vital signs by monitoring skin perfusion. Remote photoplethysmography utilizes an off-the-shelf camera and a light source to remotely detect the dynamic changes in blood volume beneath the skin and allows the extraction of blood pulsation signals. It would be desirable to provide a system for entertaining patients within a scanner whilst also allowing clinicians to take remote measurements using techniques for monitoring vital signs.

Systems which use mirrors to relay video content to patients within medical scanners already exist.

For example, US5825563 describes a device to reduce patient claustrophobia in a magnetic resonance imaging (MRI) scanner. The device described in US5825563 includes a mirror mounted on a support arm, wherein the support arm can be mounted on the patient bed of an MRI scanner.

WO2019068185 describes a device for reducing patient anxiety in medical scanners. The device described in WO2019068185 includes a mirror mounted to a support that can be attached to a patient bed.

The mirror and its support can get in the way of the patient when moving on and off the patient bed, and its presence can increase a feeling of claustrophobia. In addition, different mirror system designs are needed for different types of scanner and their associated patient supports, so that the mirror system becomes an expensive option. Thus, there remain problems with current mirror system designs.

### SUMMARY OF THE INVENTION

The invention is defined by the claims.

According to examples in accordance with an aspect of the invention, there is provided a mirror system for relaying video content to a patient on a patient support of a medical scanner, the mirror system comprising:
a flexible support arm having first and second opposite ends;
a mirror attached to the support arm; and
first and second support members, each for mounting in a position which is fixed relative to the patient support, and each comprising an end portion,
wherein the first and second ends of the support arm are each detachably and rotatably couplable to a respective one of the end portions, and when the support arm is coupled to the end portions it forms an arch.

This mirror system is for mounting to a patient support, e.g. a patient couch, of a medical scanner.

The support arm which holds the mirror is flexible so that it can be fitted to support members at different spacings. This means that the same support arm may be used with different patient supports and different support members.

The support arm forms an arch over the patient support, e.g. over the area where the patient's head will be located. The arch may be positioned to follow the inner contour of a bore of the medical scanner so that the mirror system takes up a minimal amount of space within the bore. This means that the patient can get on and off the patient support without the mirror system getting in their way.

The rotatable coupling means that the mirror position can be adjusted to give the best view of the video content (which is, for example, provided on a screen remote from the medical scanner), as well as being adjustable to make it easiest for the patient to get on and off the patient support.

The first end of the support arm and the end portion of the first support member may define a first hinge coupling and the second end of the support arm and the end portion of the second support member may define a second hinge coupling. The two hinge couplings, on opposite sides of the patient support provide a firm coupling to the support members and the support arm is easy to grip and adjust.

The position of the mirror on the flexible support arm may be adjustable. In combination with the pivoting of the support arm, this enables the mirror to be moved to a position giving the patient an optimal view of the video content.

Further, the support arm may comprise a channel and the mirror may comprise an interface configured to co-operate with the channel. This enables the position of the mirror to be adjusted by sliding it along the channel of the flexible support arm.

The system may further comprise a second mirror attached to the support arm for vital signs monitoring.

Thereby, the first mirror is used for providing entertainment to the patient and a second mirror can be for reading vital signs. For example, there may be a vital signs camera remote from the scanner, e.g. on the room wall, and the second mirror may directed to the cheek or forehead of the patient for collecting a remote PPG (rPPG) signal - from which respiration rate and/or heart rate can be extracted.

The first and second support members may be for mounting to the patient support. However, the first and second support members may also be suitable for mounting to a patient coil. That is, the first and second support members may be for mounting to a patient coil that is fixed relative to the patient support. In all cases, the support members move with the patient support.

The invention also provides a patient support system comprising:
a patient support; and
any of the mirror systems described above,
wherein the first and second support members of the mirror system are fixed relative to the patient support.

In addition, the invention also provides a medical scanner comprising:
a scanning system having a bore; and
the patient support system described above,
wherein the patient support of the patient support system extends through the bore.

The flexible support arm of the mirror system of the medical scanner forms an arch, which may follow the inner contour of the bore when the support arm is rotated to a fully open position.

The medical scanner may further comprise a screen for displaying video content, wherein the mirror system is for providing a viewing path between the patient and the screen.

The medical scanner may further comprise a second mirror attached to the support arm for vital signs monitoring, and the medical scanner then also comprises a vital signs monitoring unit for monitoring the patient using the second mirror. The vital signs monitoring unit may comprise a remote PPG camera for monitoring heart rate and/or respiration rate.

The invention also provides a patient entertainment system for using during medical scanning, with the patient on a patient support of a medical scanner, the entertainment system comprising:
a screen for displaying video content; and
any of the mirror systems described above for providing a viewing path between the patient and the screen.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a mirror system for relaying video content to a patient on a patient support of a medical scanner;
Fig. 2 shows a patient support system;
Fig. 3 shows the flexible support arm of a mirror system being manually connected to the first and second support members;
Fig. 4 shows a patient support system, wherein the first and second support members are mounted to a patient coil;
Fig. 5 shows a medical scanner according to an embodiment of the invention;
Fig. 6 shows a medical scanner comprising a screen for displaying video content;
Fig. 7 shows a medical scanner comprising a screen for displaying video content, a second mirror, and a vital signs monitoring unit; and
Fig. 8 shows a patient entertainment system.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides: a mirror system for relaying video content to a scanner patient, a patient support, a medical scanner and a patient entertainment system. Each of the patient support, medical scanner and patient entertainment system use the mirror system. The mirror system includes a flexible support arm, which is detachably and rotatably couplable to first and second support members.

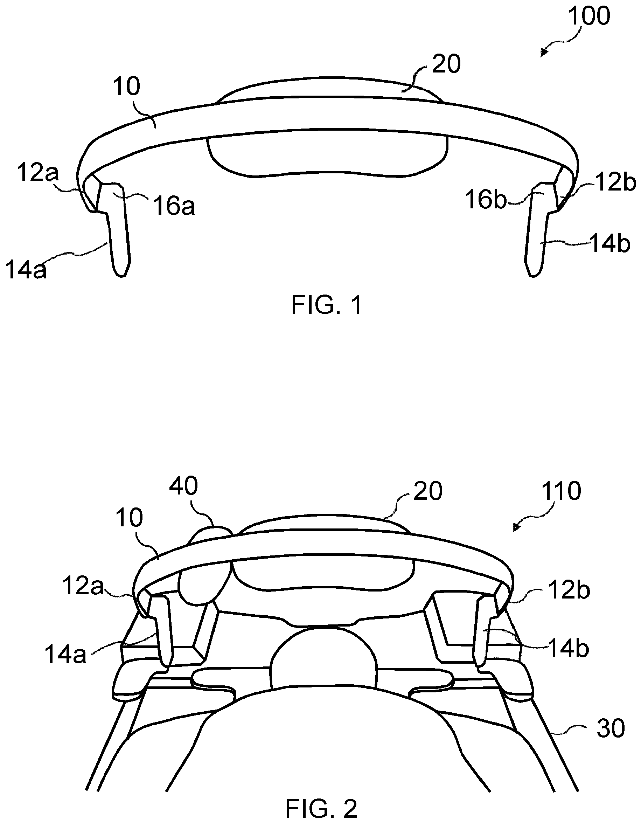

Fig. 1 shows a mirror system 100 according to an embodiment of the invention. The mirror system comprises a flexible support arm 10 having first and second opposite ends 12a, 12b. The flexible support arm 10 is attached to a mirror 20, which is positioned between the first and second ends 12a, 12b. The mirror system further comprises first and second support members 14a, 14b, each for mounting in a position which is fixed relative to a patient support. Each of the support members comprise an end portion 16a, 16b.

Fig. 1 shows the first and second ends 12a, 12b of the support arm 10 coupled to a respective one of the end portions 16a, 16b. As shown in Fig. 1, when the support arm 10 is coupled to the end portions 16a, 16b it forms an arch.

Mirror systems for directing video content to a patient on a patient support of a medical scanner already exist. However, the inventors of the present invention have realized that providing a system comprising a flexible support arm which is detachably and rotatably couplable to two support members enables the same mirror to be used on different patient supports. That is, the mirror system can be coupled to one patient support, for example a patient couch of a medical scanner, but then transferred to a different patient support with a different design from the first patient support. Thereby, the inventors of the present invention have devised a more economical mirror system that can be used on several different scanners. The scanners only need to be equipped with support members having a suitable design to enable the support arm ends 12a, 12b to be connected to them. The flexible suppport arm deisgn enables some freedom in the exact spacing between the support members.

The flexible support arm 10 may be easily locked onto the support members 14a, 14b. The flexible support arm 10 and support members 14a, 14b may be configured so that the support arm can be moved to different states (i.e. open, closed and intermediate positions). Any suitable locking and unlocking coupling may be used. For example there may be additional fastenings which are applied to couple the end portions and the ends. Alternatively, the support arm may be connectable to, and releasable from, the support members in one angular orientation (e.g. with the support arm flat against the patient support) but once rotated into an active positions (e.g. with the support arm raised over the patient support, and over head of the patient) the support arm is locked to the support members. Preferably the coupling between the support arm and the support members does not require use of any tools.

Fig. 2 shows a patient support system 110 comprising the mirror system 100 of Fig. 1 and a patient support 30. The mirror system 100 of Fig. 1 is mounted to the patient support 30 via the first and second support members 14a, 14b. In this example, the first and second support members 14a, 14b are mounted directly to the patient support. However, intermediate components between the first and second support members 14a, 14b and the patient support 30 may be provided in other embodiments. Since the arm support 10 is flexible, slight variations in the spacing or positioning of the first and second support members 14a, 14b on the patient support 30 will not prevent the arm support being coupled to the first and second support members 14a, 14b, as mentioned above. This provides the benefit that slightly different types of support members 14a, 14b and/or patient support 30 and/or positioning of the support members 14a, 14b on the patient support 30 can be used with the same support arm. This provides a more versatile system for use in the clinical setting. Although the support arm 10 is flexible, it is also designed to be sufficiently robust to keep the mirror 20 in place to withstand vibration introduced by the patient or a medical scanner.

Fig. 2 also shows how a patient can be positioned on the patient support 30 so that the mirror 20 of the patient support system 110 is positioned in their line of sight. This enables video content to be directed to the patient lying on the patient support 30, reflected from a remote video source.

Fig. 2 also shows a second mirror 40. This is used to provide a line of sight between a vital signs camera (which is remote from the scanner) and the patient, for example the cheek or forehead of the patient. The vital signs camera is for example a remote PPG (rPPG) system from which respiration rate and/or heart rate can be extracted.

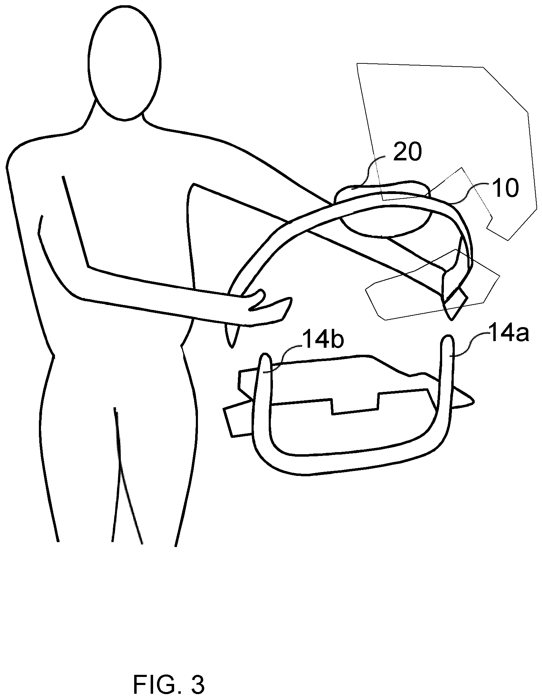

Fig. 3 shows the flexible support arm 10 of a mirror system being manually connected to the first and second support members 14a, 14b. The support arm 10 is sized so that an operator standing next to the patient support can easily couple the support arm 10 to the support members 14a, 14b.

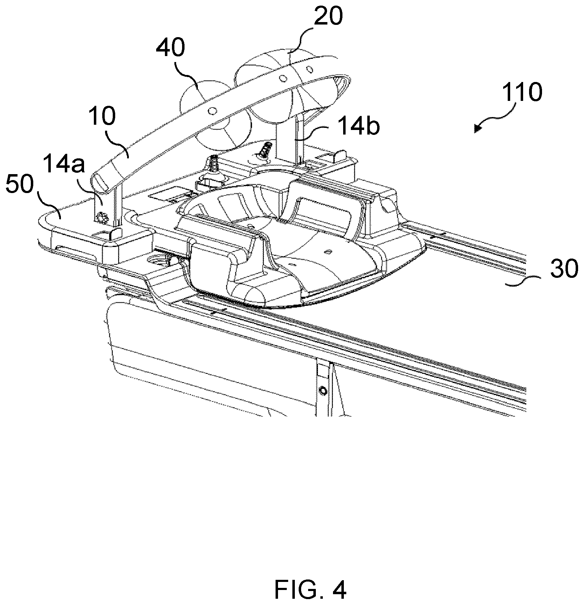

Fig. 4 shows a patient support system 120, wherein the first and second support members 14a, 14b are mounted to a patient coil 50. Although this invention is of interest for all imaging modalities, it is of particular interest to medical resonance imaging (MRI) due to the associated long scan times. Magnetic resonance imaging is a medical imaging technique that uses strong magnetic fields to generate images within the body. The skilled person will understand that a MRI system comprises several different types of coils. In particular, an MRI system will include superconducting coils to generate a static magnetic field, a set of gradient coils to generate magnetic field gradients, and radio frequency (RF) coils to transmit and receive RF pulses.

In addition, local coils may be applied to the patient within the bore of the scanner, and these local coils will be referred to as the "patient coil". The patient coil 50 may include multiple individual coil elements and the term "patient coil" should be understood accordingly.

Accordingly, it is to be understood that the first and second support members 14a, 14b can be mounted directly to the patient support 30 or they can be mounted to a patient coil 50 that is fixed relative to the patient support 30 - as shown in Fig. 4.

The support arm 10 may for example comprises a channel for adjusting the position of the mirror 20. In some embodiments the position of the second mirror 40 may also be adjusted. Either or both mirrors 20, 40 may comprise an interface (not shown) configured to co-operate with the channel. Thereby, the mirror positions can be adjusted by sliding the mirrors 20, 40 along the channel.

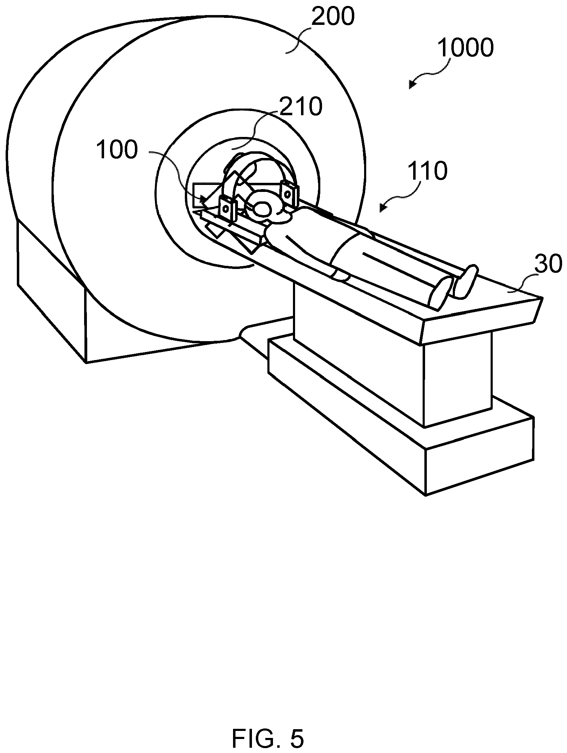

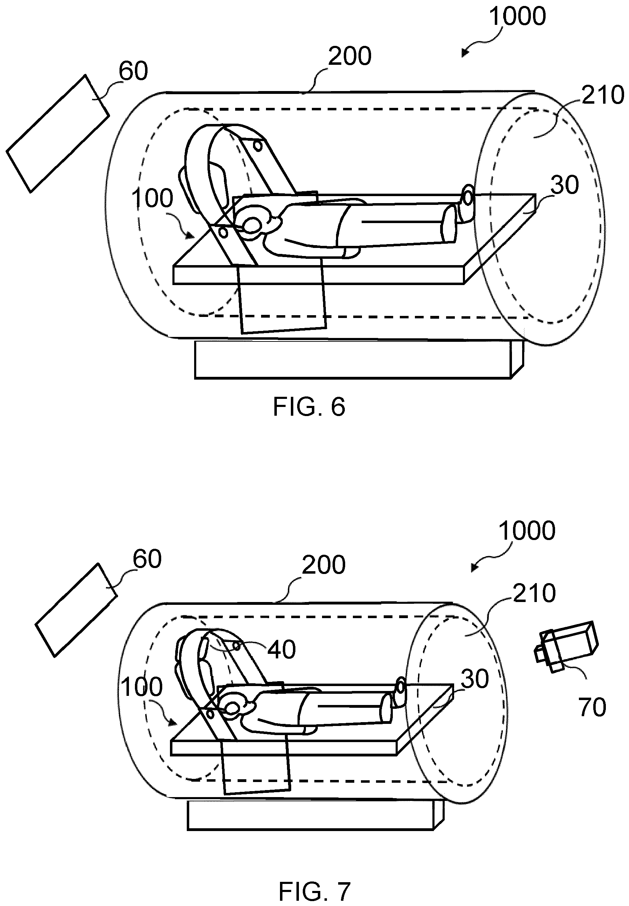

Fig. 5 shows a medical scanner 1000 comprising a scanning system 200 with a bore 210, and a patient support system 110. The patient support system 110 is shown mostly outside the bore in Fig. 6. However, the skilled person will understand that normally the patient will be positioned on the patient support 30 whilst it is outside the bore, and then the patient support 30 will be slowly moved into the bore 30 so that the patient support system 110 extends through the bore 30. In all embodiments, the patient support system comprises a mirror system 100.

Fig. 6 shows a medical scanner 1000 comprising a scanning system 200 and a screen 60 for displaying video content. The screen 60 is arranged with respect to the mirror 20 so that video content can be relayed to a patient positioned on the patient support 30.

It is desirable for the mirror system to take up the minimum possible amount of space in the bore to avoid causing any unnecessary patient claustrophobia. As shown in Fig. 7, the flexible support arm 10 forms an arch above the patient. The support arm may be positioned so that when it is in a fully open position it follows the inner contour of the bore. Preferably, the support arm may fit almost flush against the inner surface of the bore of the scanner, so that it takes up a minimum amount of space within the scanner. This arrangement means that the patient can be get on or off the patient support 30 without being obstructed by the mirror system. In addition, this has the benefit that the desired distance is maintained between the eyes of the patient and the mirror. The loop may be sized to fit a bore diameter of approximately 60 cm, or a bore diameter of approximately 70 cm.

Fig. 7 also shows a medical scanner 1000 comprising a scanning system 200 and a screen 60 for displaying video content. In addition, the medical scanner 1000 further comprises a second mirror 40 and a vital signs monitoring unit 70. The vital signs monitoring unit 70 may be positioned on the wall of the scanner room, and the second mirror 40 may be directed towards the patient's cheek or forehead. The vital signs monitoring unit 70 may comprise a rPPG camera for collecting rPPG signal from the patient. This enables clinicians to determine the heart rate and/or respiration rate of the patient. It is to be understood that other means of vital signs monitoring may be used other than rPPG monitoring.

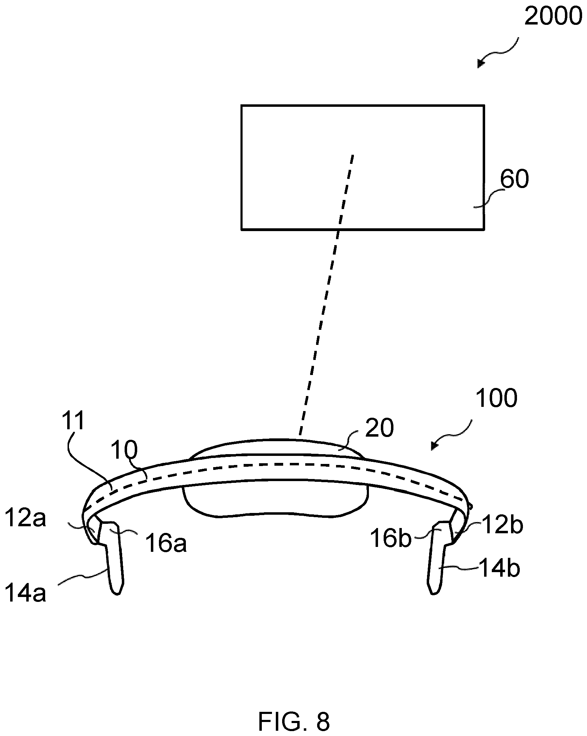

Fig. 8 shows a patient entertainment system comprising a screen 60 for displaying video content, and a mirror system 100. Fig. 8 also shows (in hidden detail) the channel 11 along which the mirror or mirrors can be slid to adjust their position along the support arm.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A mirror system (100) for relaying video content to a patient on a patient support (30) of a medical scanner (200), the mirror system comprising:
a flexible support arm (10) having first and second opposite ends (12a, 12b);
a mirror (20) attached to the support arm; and
first and second support members (14a, 14b), each for mounting in a position which is fixed relative to the patient support, and each comprising an end portion (16a, 16b),
wherein the first and second ends of the support arm are each detachably and rotatably couplable to a respective one of the end portions, and when the support arm is coupled to the end portions it forms an arch.

2. The mirror system of claim 1, wherein the first end of the support arm and the end portion of the first support member define a first hinge coupling and the second end of the support arm and the end portion of the second support member define a second hinge coupling.

3. The mirror system of claim 1 or 2, wherein the position of the mirror on the flexible support arm is adjustable.

4. The mirror system of claim 3, wherein the support arm comprises a channel (11) and the mirror further comprises an interface configured to co-operate with the channel.

5. The mirror system of any one of claims 1 to 4, comprising a second mirror (40) attached to the support arm for vital signs monitoring.

6. The mirror system of any one of claims 1 to 5, wherein the first and second support members are for mounting to the patient support.

7. The mirror system of any one of claims 1 to 5, wherein the first and second support members are for mounting to a patient coil (50).

8. A patient support system (110) comprising:
a patient support (30); and
the mirror system (100) of any one of claims 1 to 7,
wherein the first and second support members of the mirror system are fixed relative to the patient support.

9. A medical scanner (1000) comprising:
a scanning system (200) having a bore (210); and
the patient support system (110) of claim 8,
wherein the patient support of the patient support system extends through the bore.

10. The medical scanner of claim 9, wherein the flexible support arm of the mirror system forms an arch which, when the support arm is rotated to a fully open position, follows the inner contour of the bore.

11. The medical scanner of claim 9 or 10, further comprising a screen (60) for displaying video content, wherein the mirror system is for providing a viewing path between the patient and the screen.

12. The medical scanner of any one of claims 9 to 11, wherein the mirror system comprises a second mirror (40) attached to the support arm for vital signs monitoring, and wherein the medical scanner comprises a vital signs monitoring unit (70) for monitoring the patient using the second mirror.

13. The medical scanner of claim 12, wherein the vital signs monitoring unit comprises a remote PPG camera for monitoring heart rate and/or respiration rate.

14. A patient entertainment system (2000) for using during medical scanning, with the patient on a patient support of a medical scanner, the entertainment system comprising:
a screen (60) for displaying video content; and
the mirror system (100) of any one of claims 1 to 7 for providing a viewing path between the patient and the screen.
